# FASCICULE DE BREVET EUROPEEN

(11) **EP 4 105 296 B1**
(45) Date de publication et mention de la délivrance du brevet: **17.04.2024**
(21) Numéro de dépôt: 22179277.3
(22) Date de dépôt: 15.06.2022
(51) Int. Cl.: C10G 1/10, C10B 53/07, C10B 53/02, C10K 1/10, C10K 1/00, C10K 1/16, C10K 1/18, B01D 53/02, F25J 3/08, B01D 8/00, C10G 21/14, B01D 53/14, C10G 9/36, C10G 19/02, C10J 3/84

(54) **PROCÉDÉ DE TRAITEMENT D'UN FLUX GAZEUX ISSU DE LA PYROLYSE DE PLASTIQUE ET/OU DE LA PYROLYSE DE BIOMASSE, ET INSTALLATION VISANT À L INTÉGRATION DANS UN VAPOCRAQUEUR**
VERFAHREN ZUR BEHANDLUNG EINES GASSTROMS AUS KUNSTSTOFF-PYROLYSE UND/ODER BIOMASSE-PYROLYSE UND ANLAGE ZUR INTEGRATION IN EINEN STEAM CRACKER
PROCESS FOR TREATING A GAS STREAM FROM PLASTIC PYROLYSIS AND / OR BIOMASS PYROLYSIS, AND INSTALLATION FOR INTEGRATION INTO A STEAM CRACKER

(30) Priorité: 16.06.2021 FR 2106391
(43) Date de publication de la demande: 21.12.2022
(73) Titulaire: Technip Energies France, 92741 Nanterre Cedex (FR)
(72) Inventeur: REICH, VERONIQUE, 92400 COURBEVOIE (FR); SIMON, Yvon, 92400 COURBEVOIE (FR); BRAGA, Walkiria, 92400 COURBEVOIE (FR)
(74) Mandataire: McWilliams, David John

(56) Documents cités:
- FR-A1- 2 829 401
- US-A1- 2019 367 430
- US-A1- 2020 087 143

## Description

La présente invention concerne un procédé de traitement d'au moins un flux gazeux issu d'une pyrolyse de plastique et/ou de biomasse.

Les exigences de préservation de l'environnement conduisent les industriels à prévoir des filières de traitement des déchets plastiques, ou/et des filières de valorisation de la biomasse, notamment pour produire des carburants à partir de ressources renouvelables.

Ces filières utilisent la pyrolyse pour traiter les matières plastiques et/ou la biomasse afin d'obtenir des produits valorisables.

Ainsi, les gaz issus de la pyrolyse contiennent un grand nombre de composés à haute valeur ajoutée (HVC), et qui sont susceptibles d'être valorisés.

Ceci est le cas notamment de composés hydrocarbonés tels que l'éthylène, le propylène, le butadiène, le benzène, le toluène, les paraffines et l'hydrogène.

Cependant, ces gaz issus de la pyrolyse de plastique et/ou de biomasse ne sont pas directement utilisables pour être envoyés dans des craqueurs, ou dans des unités autonomes de production d'oléfines. En effet, le plastique et/ou la biomasse qui subit la pyrolyse comporte des impuretés, qui se retrouvent de manière directe ou dégradée dans les gaz de pyrolyse.

Ceci inclut par exemple des composés sulfurés tels que des mercaptans, des oxydes de soufre, ou du sulfure d'hydrogène, des gaz acides tels que de l'acide chlorhydrique ou de l'acide cyanhydrique, du monoxyde de carbone, du dioxyde de carbone, de l'oxygène et des dérivés d'oxygène, de l'ammoniac, des oxydes d'azote et autres composés dérivés d'azote ou de chlore, des métaux. Ces contaminants peuvent induire des risques de sécurité et/ou d'opérabilité dans le vapocraqueur ou l'unité autonome de récupération d'oléfines. Dans de nombreux cas, les gaz de pyrolyse ne sont donc pas valorisés pour la récupération de HVC, mais sont envoyés vers d'autres unités telles que des unités de production d'énergie.

US 9 011 578 propose aussi une solution visant à utiliser dans une unité aval de fermentation certains composés contenus dans des gaz issus de la pyrolyse de biomasse.

Avant d'introduire les gaz dans l'unité de fermentation, ceux-ci sont purifiés via un procédé de traitement qui élimine certains composés.

Un tel procédé est efficace pour obtenir des gaz compatibles avec de la fermentation, mais ne permet pas de récupérer des hydrocarbures en vue de les valoriser.

US 2020/087143 A1 décrit un procédé et une installation pour générer un gaz de synthèse. Le procédé comprend une étape d'introduction d'un flux gazeux dans une unité d'absorption cryogénique.

Un but de l'invention est de fournir un procédé qui permet de traiter de manière très efficace des gaz de pyrolyse, notamment de pyrolyse de matière plastique et/ou de biomasse, en vue de valoriser des composés que ces gaz de pyrolyse contiennent, en particulier des oléfines.

A cet effet, l'invention a pour objet un procédé de traitement selon la revendication 1.

Le procédé selon l'invention peut comprendre l'une ou plusieurs des caractéristiques des revendications 2 à 13 et la caractéristique suivante, prise(s) isolément ou suivant toute combinaison techniquement possible :
- les étapes de trempe et lavage à l'eau du flux gazeux issu de la pyrolyse, de compression, puis refroidissement du flux gazeux lavé, de lavage sous pression du flux gazeux comprimé, de passage du flux gazeux lavé dans au moins une unité d'élimination des acides, de séchage du flux gazeux appauvri en acide, de passage du flux gazeux sec dans une au moins une unité d'élimination d'impuretés, d'introduction du flux gazeux purifié dans une unité d'absorption cryogénique sont réalisées successivement dans l'ordre indiqué.

L'invention a également pour objet une installation selon la revendication 14.

L'installation selon l'invention peut comprendre l'une ou plusieurs des caractéristiques des revendications 15 et 16, prise(s) isolément ou suivant toute combinaison techniquement possible.

L'invention sera mieux comprise à la lecture de la description qui va suivre, donnée uniquement à titre d'exemple, et faite en se référant aux dessins annexés, sur lequel :
- [Fig. 1] La figure 1 est un schéma synoptique illustrant une installation de mise en oeuvre d'un premier procédé de purification selon l'invention, destiné à produire notamment une coupe d'hydrocarbures en C₂ valorisable dans un vapocraqueur ou dans une unité autonome de production d'éthylène ;
- [Fig. 2] La figure 2 est une variante d'installation destinée à la mise en oeuvre d'un deuxième procédé de purification selon l'invention

Dans tout ce qui suit, le terme « hydrocarbures en Cₙ » signifie que le nombre de carbones contenus dans l'hydrocarbure est égal à n. Le terme « hydrocarbures en Cₙ⁺ » signifie que le nombre d'atomes de carbone contenus dans l'hydrocarbure est supérieur ou égal à n.

Par exemple, le terme hydrocarbures en C₂ comprend en particulier l'éthane, l'éthylène et l'acétylène. Le terme hydrocarbures en C₂⁺ comprend des hydrocarbures ayant un nombre de carbone supérieur ou égal à 2, par exemple l'éthane, le propane, le butane, le pentane etc...

Dans tout ce qui suit, les pressions sont des pressions relatives et les compositions exprimées en pourcentages sont des pourcentages massiques, sauf indication contraire.

Par ailleurs, une même référence numérique peut désigner un courant circulant dans une conduite, ou la conduite qui les transporte.

Le terme « pression atmosphérique » désigne la pression de la masse d'air ambiante au lieu de l'installation. Cette pression est généralement comprise entre 900 mbars et 1100 mbars absolues.

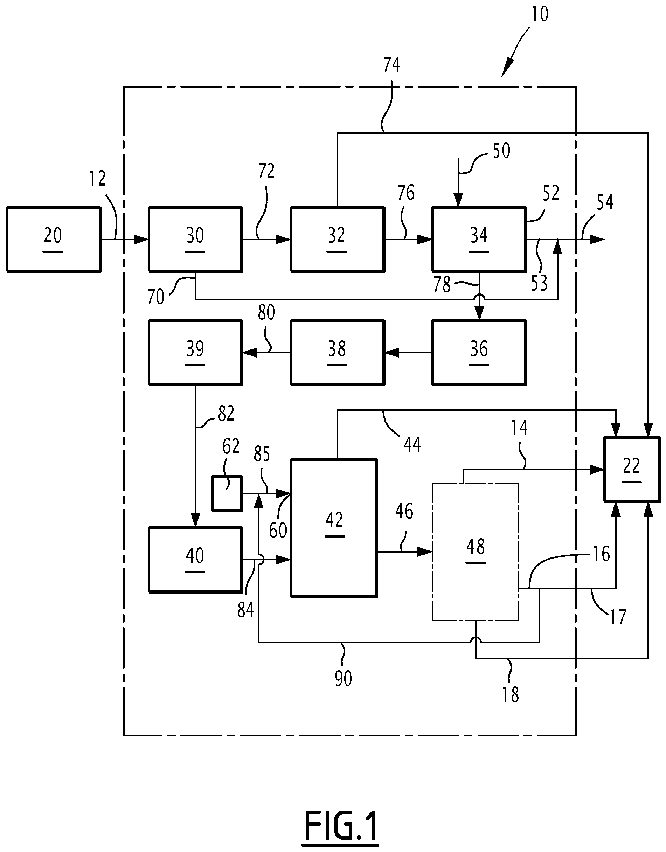

Une première installation 10 de traitement d'un flux gazeux 12 issu de la pyrolyse de matière plastique ou/et de la pyrolyse de biomasse est illustrée par la figure 1.

Cette installation 10 est destinée à la mise en oeuvre d'un premier procédé de traitement du flux gazeux 12, pour former au moins une coupe 14 d'hydrocarbures en C₂ destinée à être introduite dans une installation aval 22. L'installation aval 22 est par exemple la section de récupération d'un vapocraqueur.

Le flux gazeux 12 est traité par diverses étapes et fractionné en plusieurs coupes qui alimentent le vapocraqueur en aval des fours de craquage.

L'installation 10 selon l'invention permet l'intégration des coupes produites dans des vapocraqueurs présentant divers schémas de fractionnement : il peut s'agir d'un vapocraqueur présentant un déméthaniseur de début de production (en anglais « front-end demethanizer ») combiné à une unité d'hydrogénation aval (« back end hydrogénation »).

En variante, le vapocraqueur présente un dééthaniseur de début de production (« front-end deethanizer ») ou un dépropaniseur de début de production (« front-end depropanizer ») combiné à une unité d'hydrogénation amont (« front end hydrogénation »).

En variante encore, l'installation aval 22 est une unité autonome de récupération d'oléfines destinée à produire de l'éthylène à partir de la coupe 14 d'hydrocarbures en C₂. L'unité autonome peut également être destinée à produire du propylène à partir d'une coupe 17 d'hydrocarbures en C₃ éventuellement obtenue dans l'installation 10.

Le flux gazeux 12 est produit dans une installation 20 de pyrolyse de matière plastique et/ou de biomasse, située en amont de l'installation de traitement 10 selon l'invention.

L'installation de pyrolyse 20 est destinée notamment à effectuer la décomposition chimique de la matière plastique et/ou de la biomasse à haute température, en l'absence d'oxygène, ou dans une atmosphère pauvre en oxygène pour éviter l'oxydation et la combustion.

Par exemple, la pyrolyse est conduite dans au moins un réacteur de l'installation 20, à une température supérieure à 500 °C. La teneur en oxygène visée est la plus faible possible, la teneur résiduelle n'étant associée qu'à des phénomènes d'entrée d'air non maitrisables tels que, par exemple, ceux potentiellement induits par le fait que le déchet plastique et/ou biomasse est alimenté au réacteur par un système de convoyage à vis opérant à l'air ambiant. Cette teneur en oxygène est cependant largement inférieure à celle de l'air ambiant.

La pyrolyse produit généralement des résidus solides de pyrolyse, incluant du coke et des cendres et au moins un gaz de pyrolyse formant tout ou partie du flux gazeux 12. La pyrolyse peut également produire des liquides, notamment des liquides de pyrolyse, en particulier des huiles.

Dans le cas de la pyrolyse de matière plastique, les matières plastiques sont par exemple issues de déchets. Elles comprennent alors au moins un polymère comme du polyéthylène, du polypropylène, du polystyrène, etc., qui peut être mélangé à des résidus biogéniques tels que le papier, des résidus alimentaires ou verts, du coton, ....

Dans le cas de la pyrolyse de biomasse, la biomasse est par exemple formée de matières organiques d'origine végétale, animale, bactérienne ou fongique.

La biomasse est par exemple de la biomasse lignocellulosique, comme du bois, des résidus verts, de la paille, de la bagasse, du fourrage, de la biomasse à glucides, telle que des céréales, de la betterave sucrière, de la canne à sucre ou/et de la biomasse oléagineuse, telle que du colza, de la palme, etc.

Le flux gazeux 12 destiné à être traité dans l'installation de traitement 10 comporte par exemple, à son entrée dans l'installation 10, des composés légers tels que le méthane et au moins un composé valorisable choisi parmi des hydrocarbures en C₂, des hydrocarbures en C₃, des hydrocarbures en C₄, du benzène, du toluène, de l'hydrogène.

Par exemple, le flux gazeux 12 destiné à être traité dans l'installation 10 comprend (base humide), entre 10 % massique et 20 % massique d'hydrocarbures en C₂, entre 2 % massique et 10 % massique d'hydrocarbures en C₃, entre 1 % massique et 5 % massique d'hydrocarbures en C₄, entre 2 % massique et 10 % massique de (benzène + toluène), et entre 0,3 % massique et 4 % massique d'hydrogène

Le flux gazeux 12 comprend généralement une quantité importante d'eau, notamment une quantité supérieure à 30 % massique d'eau.

Le flux gazeux 12 étant obtenu par pyrolyse de matière plastique ou/et de biomasse, il comprend en outre divers composés ou/et impuretés qui ne sont pas utilisables ou souhaitables dans l'installation aval 22.

Le flux gazeux 12 comprend par exemple du monoxyde de carbone, du dioxyde de carbone, des oxydes d'azote, et/ou de l'oxygène et dérivés d'oxygène (alcools, aldéhydes, cétones, ...).

Il comprend par exemple entre (base humide) 2 % massique et 20 % massique de monoxyde de carbone, entre 3 % massique et 20 % massique de dioxyde de carbone.

Le flux gazeux 12 comporte éventuellement des composés azotés tels que l'azote et l'ammoniac et/ou des composés acides, tels que de l'acide chlorhydrique, et de l'acide cyanhydrique et/ou divers autres dérivés du chlore tels que les dioxines et les furanes. Il comprend par exemple entre (base sèche) 500 ppmV et 1500 ppmV d'ammoniac, entre 5000 ppmV et 150 000 ppmv d'azote, entre 50 ppmV et 5000 ppmV d'acide chlorhydrique, et entre 100 ppmV mol et 200 ppmV d'acide cyanhydrique.

Le flux gazeux 12 comprend également éventuellement des composés soufrés, tels que du sulfure d'hydrogène, des oxysulfures de carbone, des mercaptans et des thiophènes.

La teneur (base sèche) en sulfure d'hydrogène est comprise par exemple entre 200 ppmV et 1000 ppmV, celles en oxysulfures de carbone et thiophène entre 5 ppmV et 100 ppmV, et celle en mercaptans entre 1 ppmV et 100 ppmV.

La température du flux gazeux 12 est généralement supérieure à 80°C à l'entrée dans l'installation 10.

La pression du flux gazeux 12 à l'entrée dans l'installation 10 est par exemple légèrement supérieure à la pression atmosphérique, par exemple entre 1100 mbar et 3000 mbar absolues.

Tous les composés précités sont avantageusement sous forme gazeuse à l'entrée dans l'installation 10. Par entrainement liquide ou volatilité, le flux gazeux 12 peut également comprendre des métaux, de la poussière et/ou des goudrons (« tars » en anglais).

En référence à la figure 1, l'installation 10 comporte une unité de trempe/lavage 30 basse pression, une unité 32 de compression/refroidissement, et une unité de lavage 34 haute pression.

L'installation 10 comprend en outre dans cet exemple des unités 36, 38 d'élimination des acides, en particulier une unité amine 36, et une unité 38 de lavage caustique (« caustic wash » en anglais).

L'installation 10 comporte également, en aval des unités d'élimination des acides 36, 38, une unité de séchage 39, une unité 40 d'élimination des impuretés, et, en aval de l'unité 40 d'élimination des impuretés, une unité d'absorption cryogénique 42 destinée à la production d'un résidu gazeux léger 44 et d'une coupe 46 d'hydrocarbures en C₂⁺.

L'installation 10 peut en outre comporter avantageusement une unité 48 de fractionnement de la coupe 46 d'hydrocarbures en C₂⁺ pour former des coupes 14, 16, 18 d'hydrocarbures, en particulier la coupe 14 d'hydrocarbures en C₂ destinée à l'installation aval 22, une coupe 16 d'hydrocarbures en C₃, destinée à être au moins partiellement recyclée dans l'unité d'absorption cryogénique 42 et une coupe 18 d'hydrocarbures en C₄⁺.

L'unité de trempe/lavage 30 comporte par exemple au moins une capacité de trempe/lavage, et au moins un injecteur d'eau sous forme liquide dans la capacité. L'eau injectée est destinée à refroidir le flux gazeux 12 et à le condenser partiellement pour récupérer une phase aqueuse 70.

L'unité de compression/refroidissement 32 comporte au moins un compresseur, le plus souvent multi-étagé, doté de refroidissement inter-étages, notamment d'un refroidissement à eau ou à air.

L'unité de lavage sous pression 34 comporte avantageusement au moins une entrée 50 d'injection d'eau sous pression, au moins une chambre de lavage 52 destinée à recevoir l'eau sous pression et au moins une sortie 53 d'évacuation d'eau sous pression hors de la chambre 52.

L'unité amine 36 comporte classiquement un absorbeur et un régénérateur. Dans l'absorbeur, un flux descendant d'amines rencontre un flux ascendant gazeux, et reçoit les gaz acides que le flux gazeux contient. Le flux gazeux adouci sort de l'absorbeur avec une teneur réduite en acides. Le flux d'amines enrichi en acides coule est évacué vers le régénérateur. Un ballon de flash est usuellement installé entre l'absorbeur et le régénérateur. Afin de limiter les pertes en hydrocarbures, le flux gazeux issus de cette étape peut être recyclé à l'unité de compression 32.

Dans le régénérateur, le flux d'amines enrichi en acides est chauffé et est convoyé vers un évaporateur pour évacuer des vapeurs riches en acides. Le flux d'amines redevenu pauvre en acides est refroidi par le flux d'amines enrichi issue de l'absorbeur, et est alors renvoyé dans l'absorbeur.

L'unité de lavage caustique 38 comporte au moins une capacité munie d'une entrée d'alimentation de solution de soude pour permettre le lavage du flux gazeux par de l'hydroxyde de sodium.

L'installation de séchage 39 comporte usuellement un refroidisseur au propylène, suivi d'un séparateur liquide/vapeur dont le flux de tête est passé au travers de lits de tamis moléculaire, notamment de 3 Angström. L'installation de séchage 39 est propre à éliminer l'eau présente dans le flux gazeux tout en ne piégeant pas les oléfines présents dans le flux gazeux. L'unité 39 peut également comporter une fonction d'abattement du mercure.

L'unité d'élimination des impuretés 40 est propre à fournir une protection additionnelle quant à l'élimination notamment de l'ammoniac et autres composés azotés, des composés chlorés, des composés soufrés, des alcools, de l'eau. Elle comporte par exemple des adsorbants à lits actifs, notamment des lits métalliques actifs tels que des lits d'alumine active ou d'autres adsorbants mixtes.

L'unité d'absorption cryogénique 42 comporte au moins une colonne d'absorption cryogénique, une entrée 60 d'introduction de solvant, et une source 62 de solvant frais.

La source 62 est propre à fournir un appoint de solvant.

Le solvant est constitué par exemple d'hydrocarbures liquides, en particulier d'hydrocarbures en C₃⁺ liquides, notamment de propane liquide.

Par « cryogénique », on entend que la température du solvant 85 et du flux gazeux 84 alimentant la colonne d'absorption cryogénique est inférieure à - 20°C, notamment comprise entre - 30°C et - 40 °C.

La colonne d'absorption destinée à mettre en contact le flux gazeux avec le solvant peut être dotée de plateaux ou de garnissage.

L'unité de fractionnement 48 qui est optionnelle comporte au moins une colonne de distillation, en particulier au moins une première colonne de distillation propre à séparer en tête la coupe 14 d'hydrocarbures en C₂ d'au moins une coupe d'hydrocarbures en C₃⁺, produite en pied.

Elle comprend avantageusement une deuxième colonne de distillation, propre à traiter la coupe d'hydrocarbures en C₃⁺, pour former la coupe 16 d'hydrocarbures en C₃, et la coupe 18 d'hydrocarbures en C₄⁺ récupérée en pied de la deuxième colonne de distillation.

Un premier procédé de traitement d'un flux gazeux 12 issu de l'installation de pyrolyse 20 va maintenant être décrit.

Initialement, le flux gazeux 12 présentant la composition telle que décrite plus haut, est produit par l'installation de pyrolyse 20. Il présente généralement une température supérieure à 80° C, et une pression comprise entre 1100 mbar et 3000 mbar absolues.

Comme indiqué plus haut, le flux gazeux 12 contient généralement une quantité importante de vapeur d'eau, notamment plus de 30% massique d'eau.

Le flux gazeux 12 est introduit dans l'unité de trempe/lavage 30. Il est mis en contact direct avec de l'eau de lavage à basse pression, pour réaliser une trempe du flux gazeux 20 et condenser au moins partiellement l'eau contenue dans le flux gazeux 20.

La pression opératoire dans la colonne de trempe/lavage à l'eau 30 est inférieure à 2 bar, notamment à la pression du flux gazeux 12 duquel est déduite la perte de charge en ligne, soit une pression très légèrement supérieure à la pression atmosphérique.

La température de l'eau de lavage introduite dans l'unité de trempe/lavage 30 est fixée par les conditions de site. Ainsi, si la température d'eau de refroidissement disponible sur le site est de 35°C, une température typique pour l'eau de lavage est de 37 à 40°C.

En pied de la colonne de trempe/lavage à l'eau 30, une phase aqueuse correspondant à l'eau de lavage et une partie de l'eau contenue dans le flux gazeux 20 est ainsi récupérée.

L'eau de lavage est renvoyée en tête de colonne après avoir été refroidie, tandis que la partie correspondant à l'eau condensée est extraite. Elle forme une phase aqueuse 70 qui est donc récupérée séparément du flux gazeux refroidi et lavé 72 formé dans l'unité de trempe/lavage 30.

Avantageusement, la phase aqueuse 70 comprend plus de 85 % de l'eau contenue dans le flux gazeux 12 introduit dans l'unité de trempe/lavage. Le flux gazeux refroidi et lavé 72 comporte typiquement moins de 10% massique d'eau.

Le lavage à travers la colonne de lavage/trempe 30 permet l'abattement des composés solubles dans l'eau tels que l'ammoniac, les alcools ou l'acide chlorhydrique. Avantageusement, l'eau de lavage peut être acidifiée pour amplifier cette fonction. Le lavage permet aussi d'abattre les potentiels métaux, poussière et/ou goudrons (« tars ») présents dans le flux 12.

Le flux gazeux lavé 72 est récupéré à une température fixée par les conditions de site. Ainsi, si la température d'eau de refroidissement disponible sur le site est de 35°C, une température typique pour le flux gazeux lavé 72 est de 40°C à 45°C. Le flux gazeux lavé 72 est introduit alors dans l'unité de compression/refroidissement 32, pour être comprimé à une pression supérieure à 10 bar, notamment comprise entre 20 bar et 30 bar.

A chaque étage de compression, le flux gazeux 72 est refroidi dans un réfrigérant, à une température fixée par les conditions de site, par exemple 40°C. Avantageusement, un refroidisseur final au propylène réfrigérant peut être implémenté pour diminuer la température du flux 76 par exemple entre 15°C et 20°C et optimiser ainsi davantage la récupération de condensats.

Un condensat d'hydrocarbures 74 est récupéré. Ce courant contient notamment (base sèche) du benzène (par exemple entre 60 % massique et 80 % massique), du toluène (par exemple entre 15 % massique et 25 % massique), des hydrocarbures en C4 (par exemple entre 5 % massique et 15 % massique), des hydrocarbures en C3 et plus légers (typiquement moins de 5% massique), des hydrocarbures en C5 (typiquement moins de 2% massique), ainsi que des traces de gaz dissous, de l'eau, et des impuretés telles que les composés soufrés ou autres.

La coupe 74 est avantageusement envoyée vers l'installation aval 22, pour permettre l'extraction des composés ayant une valeur commerciale, par exemple le benzène, le toluène et le butadiène.

Un flux gazeux comprimé 76 est formé à la sortie de l'unité de compression/refroidissement 32. Le flux gazeux comprimé 76 est introduit dans l'unité de lavage haute pression 34. Il est mis en contact avec de l'eau de lavage sous pression provenant de l'entrée 50, à une pression supérieure à 10 bar (avantageusement supérieure à 20 bar) et à une température avantageusement supérieure à 45°C.

Le lavage du flux gazeux comprimé 76 réduit la quantité d'acide cyanhydrique présente dans le flux gazeux comprimé 76. Contrairement au lavage basse pression 30 qui recircule l'eau, ce lavage haute pression est avantageusement réalisé avec de l'eau circulant en une seule passe (« one-through » en anglais) de telle sorte à maximiser l'élimination de l'acide cyanhydrique. Avantageusement, plus de 75 % de l'acide cyanhydrique contenu dans le flux gazeux comprimé 76 est récupéré dans l'eau sous pression à la sortie 53.

Le flux gazeux lavé 78 produit à la sortie de l'unité de lavage haute pression 34 présente ainsi une teneur en acide cyanhydrique qui dépend de la quantité présente dans le flux gazeux 12, donc du type de déchet alimentant l'unité de pyrolyse 20. Par exemple, la teneur est comprise entre 50 ppmV et 500 ppmV.

Le lavage à haute pression permet également d'atteindre des spécifications plus poussées sur les impuretés déjà abattues dans le lavage basse pression 30. Les teneurs en ammoniac et acide chlorhydrique dans le flux 78 sont par exemple inférieures à respectivement à 2 ppmV et 1 ppmV.

Le flux gazeux lavé 78 est ensuite passé successivement dans chaque unité d'élimination des acides 36, 38.

Dans ces unités, il est mis en contact successivement avec le flux d'amine, et avec l'hydroxyde de sodium, pour éliminer notamment le dioxyde de carbone et le sulfure d'hydrogène contenus dans le flux gazeux lavé 78.

Des coûts opératoires de l'unité aux amines peuvent notamment être engendrés par les produits de dégradation de l'amine qui sont favorisés par la présence dans le flux 78 de composés oxygénés et d'acide cyanhydrique résiduel. Pour réduire ces coûts, l'unité d'amine 36 peut comporter des spécificités comme la présence d'un purificateur (« reclaimer » en anglais) et/ou de systèmes à lit échangeurs d'ions.

Avantageusement, le dioxyde de carbone contenu et le sulfure d'hydrogène contenus dans le flux gazeux lavé 78 sont éliminés dans les unités 36, 38 de sorte à ce que le flux gazeux appauvri en acides 80 produit en sortie des unités 36, 38 comporte de préférence moins de 0,5 ppmV de dioxyde de carbone, et de préférence moins de 0,1 ppmV de sulfure d'hydrogène.

Avantageusement, le lavage aux amines et à la soude des unités 36 et 38 permet également l'abattement de l'acide cyanhydrique résiduel et l'abattement partiel de certains composés soufrés tels que les mercaptans.

Ensuite, le flux gazeux appauvri en acides 80 est introduit dans l'unité de séchage 39, où sa teneur en eau est réduite. Un flux gazeux sec 82 est ainsi formé.

Le flux gazeux sec 82 présente avantageusement une teneur en eau inférieur à 1 ppm en masse. L'unité 39 peut également avantageusement comporter une fonction d'abattement du mercure jusqu'à des teneurs typiques en dessous de 10 ng/NM³.

Puis, le flux gazeux sec 82 est introduit dans l'unité d'élimination des impuretés 40, pour permettre une protection additionnelle quant à l'élimination notamment de l'ammoniac et autres composés azotés, et/ou des composés chlorés, et/ou des composés soufrés (notamment l'oxysulfure d'hydrogène et des mercaptans), et/ou des composés oxygénés (notamment les alcools) pour former un flux gazeux purifié 84. L'unité 40 peut également permettre l'abattement d'autres impuretés (par exemple le silicone). Elle comporte par exemple des adsorbants à lits actifs, notamment des lits métalliques actifs tels que des lits d'alumine active ou autres adsorbants mixtes positionnés en série.

La teneur totale en impuretés dans le flux gazeux purifié 84 est de préférence inférieure à 1 ppm massique.

Le flux gazeux purifié 84 est ensuite introduit dans l'unité d'absorption cryogénique 42. Il est d'abord refroidi à une température cryogénique, puis mis en contact avec un solvant hydrocarboné froid 85 dans une colonne d'absorption cryogénique. La colonne d'absorption destinée à mettre en contact le flux gazeux avec le solvant peut être dotée de plateaux ou de garnissage.

Le solvant hydrocarboné 85 est refroidi à une température cryogénique, puis est introduit par l'entrée 60 dans la colonne d'absorption. Le solvant hydrocarboné 85 est formé de solvant provenant de la source d'appoint 62 et éventuellement, de solvant recyclé provenant de l'unité de fractionnement 48.

Comme indiqué plus haut, le solvant hydrocarboné 85 comporte des hydrocarbures en C₃ et/ou des hydrocarbures en C₄₊. De préférence, il comporte plus de 50% molaire d'hydrocarbures en C₃, et avantageusement plus de 90% molaire d'hydrocarbures en C₃, en particulier du propane.

La température du solvant cryogénique hydrocarboné 85 est ici inférieure à -20°C, et avantageusement comprise entre - 30°C et - 40 °C. La pression de la colonne d'absorption cryogénique est usuellement comprise entre 15 bar et 25 bar.

Le solvant hydrocarboné 85 est avantageusement introduit en tête de la colonne d'absorption, et circule à contre-courant du flux gazeux purifié 84 introduit dans l'unité d'absorption cryogénique 42, au pied de la colonne d'absorption.

Ainsi, les hydrocarbures en C₂⁺ contenus dans le flux gazeux purifié 84 sont collectés par le solvant hydrocarboné 85 et sont récupérées sous forme d'une coupe 46 d'hydrocarbures en C2+ au pied de la colonne d'absorption. Au contraire, les gaz résiduels forment le résidu gazeux léger 44 récupéré en tête de l'unité d'absorption cryogénique.

La colonne d'absorption est par exemple rebouillie à la vapeur basse pression (par exemple à une pression inférieure à 5 bar de sorte à maintenir la spécification de méthane dans la coupe 46 d'hydrocarbures en C2+.

De manière optionnelle, la colonne d'absorption peut être dotée d'un ou plusieurs reflux circulants intermédiaire pour améliorer l'efficacité de l'absorption des composés à haute valeur ajoutés dans le solvant. Usuellement, les reflux circulants intermédiaires sont refroidis à une température avantageusement comprise entre - 30°C et - 40 °C avant d'être réinjectés dans la colonne.

Afin de limiter les pertes en solvant dans le flux de tête de la colonne d'absorption, celui-ci est usuellement envoyé vers une étape de condensation partielle. Le liquide formé est alors renvoyé dans le flux de solvant 85, tandis que le résidu gazeux léger 44 (flux « fuel gas ») est détendu, réchauffé à la température ambiante et renvoyé dans le réseau de fuel du vapocraqueur.

Avantageusement, plusieurs intégrations thermiques citées dans ce procédé peuvent être combinées dans un seul équipement cryogénique à plaque en aluminium brasées (dite « boite froide »), ce qui optimise le coût de l'unité, ainsi que la récupération d'énergie. Par exemple, le refroidissement du flux gazeux purifié 84, le refroidissement du solvant 85, le réchauffage du résidu gazeux léger 44, le refroidissement du (ou des) reflux circulant(s) de la colonne d'absorption cryogénique, et/ou la condensation partielle de la colonne d'absorption cryogénique peuvent être réalisés dans le même équipement.

La coupe d'hydrocarbures en C₂⁺ 46 contient de préférence plus de 99,5 % des hydrocarbures en C₂ contenus dans le flux gazeux purifié 84.

Le résidu gazeux léger 44 contient avantageusement du gaz combustible, en particulier du méthane, de l'hydrogène, ainsi que les autres composés légers du flux gazeux purifié 84 tels que le monoxyde de carbone et l'azote. Il contient des composés hydrocarbonés provenant principalement du solvant 85, ainsi que les hydrocarbures en C2 présents dans le flux gazeux purifié 84 non récupérés dans la coupe 46. Enfin, le résidu léger 44 peut contenir également les impuretés légères de la coupe 46 telles que par exemple de l'oxyde d'azote, de l'oxygène et/ou de l'argon ainsi que par volatilité d'autres impuretés telles que par exemple des composés soufrés légers ou de l'ammoniac.

En particulier, le résidu gazeux léger 44 comporte l'oxyde d'azote et l'oxygène contenus dans le flux gazeux purifié 84. Ainsi, la teneur de ces composés dans la coupe 46 d'hydrocarbures en C2+ est en dessous des limites de détection. Par ailleurs, cette coupe 46 présente de très faibles quantités de légers. Par exemple, la teneur en méthane rapportée à l'éthylène peut être inférieure ou égale à 500 ppm molaire. Ceci garantit la spécification du méthane dans l'éthylène sans fractionnement additionnel.

De la sorte, la coupe d'hydrocarbures en C₂⁺ 46 (ainsi que par extension les coupes issues de celle-ci, par exemple les coupes 14, 17, 18) sont aptes à être intégrées dans une section de fractionnement de vapocraqueur en évitant les dangers liés à la présence d'oxydes d'azote.

En effet, lorsque des gaz contenant de manière combinée de l'oxyde d'azote, de l'oxygène des diènes et/ou de l'ammoniaque sont portés à basse température, en particulier à des températures inférieures à -100°C, des gommes ou sels de NOx peuvent être formés. Ces composés présentent un risque d'explosion lorsqu'ils sont portés à température ambiante. Le flux gazeux purifié 84 ne peut donc pas être porté à basse température, en particulier aux températures usuellement rencontrées dans les bouts froids des boites froides de vapocraqueur. En particulier, le mélange du flux gazeux purifié 84 avec des gaz craqués issus d'un vapocraqueur dont le schéma présente un déméthaniseur de début de production (en anglais « front-end demethanizer ») ne permettrait pas de garantir la sécurité de l'installation, puisque le flux combiné serait porté à des températures inférieures à - 100°C avant d'alimenter le déméthaniseur.

La température la plus froide rencontrée dans l'unité d'absorption cryogénique 42 est limitée aux températures pouvant être atteintes lorsque le propylène utilisé comme réfrigérant. Cette température est toujours supérieure ou égale à la température d'équilibre du propylène à la pression atmosphérique (-47°C). Ceci supprime le risque d'explosion de ces gommes ou sels lorsqu'ils se réchauffent.

Le procédé selon l'invention supprime le monoxyde d'azote, l'oxygène et l'ammoniac de manière très efficace, notamment dans le cas où il n'est pas possible de mettre en oeuvre une élimination des oxydes d'azote et de l'oxygène en utilisant un catalyseur de type déoxygénation, en raison du contenu important en monoxyde de carbone et en composé dioléfinique du flux gazeux 12.

En outre, le procédé selon l'invention est très utile pour l'intégration du flux 84 dans un vapocraqueur dont le schéma est un dééthaniseur de début de production (« front-end deethanizer ») ou un dépropaniseur de début de production (« front-end depropanizer ») combiné à une unité d'hydrogénation amont (« front end hydrogénation »). Le problème décrit ci-dessus lié à l'oxyde d'azote est minimisé puisque dans ce schéma seule la partie légère ayant subie une hydrogénation est portée à des températures inférieures à -100°C. Cependant, dans ce schéma, le convertisseur d'acétylène est très sensible à des emballements lorsque se produisent des fluctuations de contenu en monoxyde de carbone.

La coupe 46 d'hydrocarbures en C₂⁺ (ainsi que par extension les coupes issues de celle-ci, par exemple les coupes 14, 17, 18) étant dépourvues de monoxyde de carbone, le risque d'un tel emballement est limité, et l'intégration de ces coupes en amont du convertisseur d'hydrogénation est rendu sûre.

Par ailleurs, comme discuté ci-dessus, le procédé selon l'invention garantit le respect des spécifications en méthane de l'éthylène, tout en conservant des conditions cryogéniques peu exigeantes par l'utilisation d'une simple unité d'absorption cryogénique 42, ce qui est avantageux, notamment dans le cas où l'installation aval 22 est une unité de récupération d'oléfines autonome.

La coupe 46 d'hydrocarbures en C₂⁺ produite dans l'unité d'absorption cryogénique 42 est introduite dans l'unité de fractionnement 48, lorsque celle-ci est présente.

Dans la première colonne de distillation de l'unité de fractionnement 48, la coupe 14 d'hydrocarbures en C₂ est récupérée en tête.

Cette coupe 14 comporte plus de 99 % des hydrocarbures en C₂ compris dans la coupe 46 d'hydrocarbures en C₂⁺. Elle comprend usuellement plus de 99,5 % molaire d'hydrocarbures en C₂, notamment de l'éthylène.

La coupe 14 est ensuite introduite dans l'installation aval 22, comme décrit plus haut

Un courant d'hydrocarbures en C₃⁺ est formé en pied de la première colonne de distillation. Ce courant est de préférence introduit dans une deuxième colonne de distillation, pour séparer la coupe 16 d'hydrocarbures en Csde la coupe 18 d'hydrocarbures en C₄⁺.

La coupe 16 d'hydrocarbures en C₃ comporte généralement les hydrocarbures en C₃ présents dans le flux gazeux purifié 84 et ceux introduits dans le solvant 85. Un courant 90 de recyclage de solvant est formé à partir d'au moins une partie de la coupe 16 d'hydrocarbures en C₃.

Le courant de recyclage 90 est alors recyclé vers l'entrée 60 d'introduction de solvant, pour constituer une partie du solvant 85 introduit dans l'unité d'absorption cryogénique 42. Ceci limite les appoints de solvant 85 à partir de la source 62. Par ailleurs la partie résiduelle 17 du flux 16 est ensuite introduite dans l'installation aval 22 pour permettre notamment la récupération de propylène.

Une coupe 18 d'hydrocarbures en C₄⁺ est récupérée en pied de la deuxième colonne de distillation. Elle est avantageusement envoyée vers l'installation aval 22, pour permettre l'extraction des composés ayant une valeur commerciale, par exemple le butadiène, le benzène et le toluène.

Le procédé selon l'invention élimine donc de manière efficace les contaminants présents dans le flux gazeux 12 issus de la pyrolyse de plastique et/ou de biomasse qui pourraient compromettre son intégration dans une installation avale type vapocraqueur (ou autre unité de récupération d'oléfines). En effet, ils comprennent souvent des composés engendrant des risques pour la sécurité de ces installations, pour la qualité des produits finis et/ou pour l'empoisonnement des catalyseurs présents dans ces installations.

Il est ainsi possible de produire un flux gazeux purifié 84 dépourvu de ces contaminants. En outre, la séparation du résidu gazeux léger 44 produit un résidu qui peut être intégré dans des procédés aval, par exemple comme combustible, ou pour de la récupération d'hydrogène.

Par exemple, le monoxyde d'azote présent dans le flux gazeux 12 est éliminé efficacement dans l'unité d'absorption cryogénique 42, limitant les risques de formation de gommes ou de sels d'oxyde d'azote dans des étapes ultérieures.

L'élimination du monoxyde de carbone dans le résidu gazeux 44 évite également les fluctuations de contenu en monoxyde de carbone dans la coupe 46 d'hydrocarbures en C₂⁺ (ainsi que par extension dans les coupes issues de celle-ci, par exemple les coupes 14, 17, 18), pour réduire le risque d'emballement de réaction dans les réacteurs d'hydrogénation d'acétylène de certaines unités de vapocraqueur (ou unité de récupération d'oléfines autonome).

Ainsi, la coupe 46 d'hydrocarbures en C₂⁺ produite dans l'installation 10 (ainsi que par extension les coupes issues de celle-ci, par exemple les coupes 14, 17, 18), est apte à être utilisée directement dans des unités de vapocraquage, ou peut être valorisée sous forme de produit fini, dans une unité de production d'oléfines autonome.

La description du nombre et de l'arrangement des colonnes de fractionnement de l'unité 48, ainsi que du piquage du flux de recyclage 90 discutée ci-dessus correspond à un cas où le solvant frais utilisé comporte des hydrocarbures en C3 de manière majoritaire. Selon le solvant utilisé et la taille de l'unité, des variantes pourraient être envisagées.

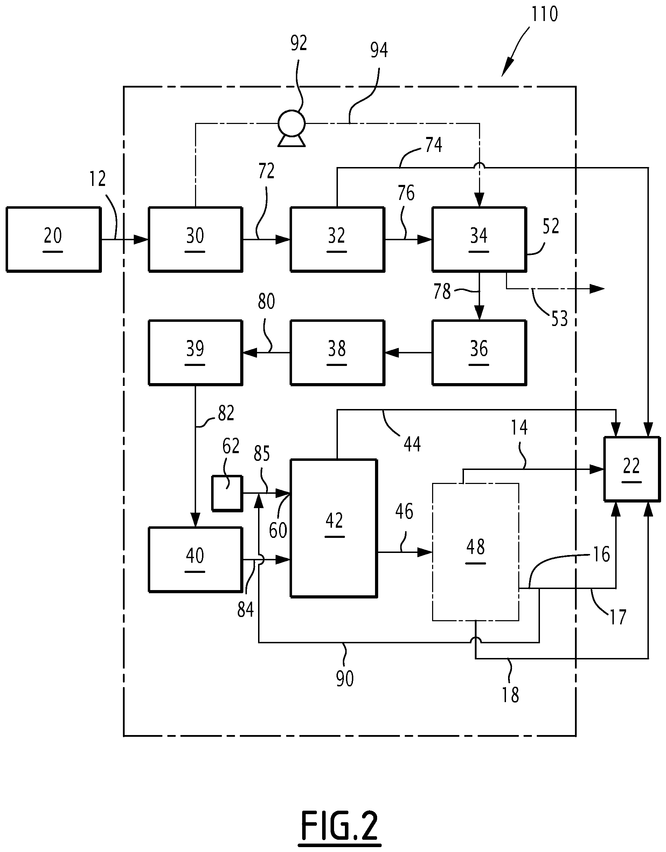

Dans la variante d'installation 110 illustrée par la figure 2, la phase aqueuse 70 issue de l'unité de trempe/lavage 30 est récupérée et est envoyée à un organe de compression 92, qui est ici une pompe.

La phase aqueuse 70 est pompée jusqu'à la pression requise pour effectuer le lavage à haute pression, pression avantageusement supérieure à 20 bars, pour former une phase aqueuse comprimée 94.

La phase aqueuse comprimée 94 est alors introduite dans la chambre de lavage haute pression 52, dans l'unité de lavage sous pression 34. Elle est mise en contact avec le flux gazeux comprimé 76, pour permettre le lavage haute pression du flux gazeux comprimé 76.

Comme l'installation 10 de la figure 1, le lavage haute pression réduit très efficacement la quantité d'acide cyanhydrique présente dans le flux gazeux comprimé 76 jusqu'à une teneur comprise par exemple entre 50 ppmV et 500 ppmV. Cette efficacité est permise grâce à circulation de l'eau en une seule passe (« one-through » en anglais). Cependant, le débit d'eau requis pour maximiser l'efficacité du lavage est conséquent et l'utilisation de la phase aqueuse 70, qui correspond essentiellement l'eau du flux gazeux 12 condensée dans l'unité de trempe/lavage 30, limite voire supprime l'ajout d'eau douce (eau de procédé, « boiler feed water » en anglais ») supplémentaire. Ainsi, dans l'installation 10 de la figure 1, l'eau de lavage 50 est de l'eau de procédé devant être amenée en limite d'unité et qui donc représentera un coût opératoire, tandis que dans l'installation 110 la figure 2, l'eau de lavage correspond à phase aqueuse 70 produite à l'intérieur de l'unité.

Dans l'installation 10 de la figure 1, le débit d'eau 54 à renvoyer vers l'unité extérieure de traitement des eaux correspond au débit combiné de la phase aqueuse 70 et de l'eau collectée à la sortie 53, tandis que dans l'installation 110 de la figure 2 il est fortement diminué et ne correspond qu'au débit de l'eau collectée à la sortie 53. Cet arrangement réduit les coûts d'opération de l'installation 10 et de l'installation de traitement d'eau aval.

## Revendications

1. Procédé de traitement d'au moins un flux gazeux (12) issu d'une pyrolyse de plastique et/ou de biomasse, **caractérisé par** les étapes suivantes :
- trempe et lavage à l'eau du flux gazeux issu de la pyrolyse (12) de plastique et/ou de biomasse dans une unité (30) de trempe/lavage et séparation d'une phase aqueuse (70) et d'un flux gazeux lavé (72);
- compression, puis refroidissement du flux gazeux lavé (72) pour former un flux gazeux comprimé (76) ;
- lavage sous pression du flux gazeux comprimé (76) pour former un flux gazeux lavé (78);
- passage du flux gazeux lavé (78) dans au moins une unité (36, 38) d'élimination des acides pour former un flux gazeux appauvri en acide (80) ;
- séchage du flux gazeux appauvri en acide (80) pour former un flux gazeux sec (82);
- passage du flux gazeux sec (82) dans une au moins une unité d'élimination d'impuretés (40) pour former un flux gazeux purifié (84);
- introduction du flux gazeux purifié (84) dans une unité d'absorption cryogénique (42) et alimentation de l'unité d'absorption cryogénique (42) avec un solvant cryogénique hydrocarboné (85) pour obtenir, à une première sortie de l'unité d'absorption cryogénique (42), un résidu gazeux léger (44), et à une deuxième sortie de l'unité d'absorption cryogénique (42), une coupe (46) d'hydrocarbures en C₂⁺.

2. Procédé selon la revendication 1, dans lequel le flux gazeux issu de la pyrolyse (12) contient des hydrocarbures en C₂⁺, et des composés légers tels que de l'hydrogène, et du méthane.

3. Procédé selon l'une quelconque des revendications 1 ou 2, dans lequel le flux gazeux issu de la pyrolyse (12) contient du monoxyde de carbone, du dioxyde de carbone, des oxydes d'azote, et/ou de l'oxygène et dérivés d'oxygène notamment des alcools, des aldéhydes ou/et des cétones, des composés azotés tels que l'azote et l'ammoniac et/ou des composés acides, tels que de l'acide chlorhydrique, et de l'acide cyanhydrique, des dérivés du chlore, et/ou des composés soufrés, notamment des mercaptans, des oxysulfures de carbone, et du sulfure d'hydrogène.

4. Procédé selon l'une quelconque des revendications précédentes, dans lequel le solvant cryogénique hydrocarboné (85) comporte des hydrocarbures en C₃ et/ou en C₄+, en particulier des hydrocarbures en C₃.

5. Procédé selon l'une quelconque des revendications précédentes, comportant une étape de séparation de la coupe (46) d'hydrocarbures en C₂⁺ en une coupe (14) d'hydrocarbures en C₂, et en au moins une coupe (16, 18) d'hydrocarbures en C₃⁺, au moins une partie de ladite coupe (16, 18) d'hydrocarbures en C₃⁺ étant recyclée dans l'unité d'absorption cryogénique (42).

6. Procédé selon la revendication 5, dans lequel l'étape de séparation comporte la production d'une coupe (16) d'hydrocarbures en C₃ et d'une coupe (18) d'hydrocarbures en C₄⁺, au moins une partie de la coupe (16) d'hydrocarbures en C₃ étant recyclée dans l'unité d'absorption cryogénique (42).

7. Procédé selon l'une quelconque des revendications précédentes, dans lequel le résidu gazeux léger (44) contient du gaz combustible, en particulier du méthane, de l'hydrogène, ainsi que des composés légers du flux gazeux purifié (84), tels que le monoxyde de carbone, de l'azote, du monoxyde d'azote, et/ou de l'oxygène.

8. Procédé selon l'une quelconque des revendications précédentes, dans lequel le résidu gazeux léger (44) est destiné à être convoyé dans une unité de craquage en particulier dans le réseau de gaz combustible d'un vapocraqueur et/ou est destiné à constituer un gaz carburant.

9. Procédé selon l'une quelconque des revendications précédentes, dans lequel au moins une unité d'élimination des acides comporte une unité amine (36) ou/et une unité de lavage caustique (38), le flux gazeux comprimé (76) passant dans l'unité amine (36) ou/et dans l'unité de lavage caustique (38).

10. Procédé selon l'une quelconque des revendications précédentes, dans lequel l'unité d'élimination d'impuretés (40) comporte au moins un lit métallique activé, notamment un lit d'alumine activé ou un adsorbant mixte ; les composés soufrés, notamment l'oxysulfure d'hydrogène et les mercaptans, et/ou l'ammoniac, les composés azotés, les composés chlorés, les composés oxygénés, notamment les alcools, et/ou les silicones contenus dans le flux gazeux sec (82) étant retenus sur le au moins un lit métallique activé.

11. Procédé selon l'une quelconque des revendications précédentes, dans lequel au moins une partie de la coupe (46) d'hydrocarbures en C₂⁺ est introduite dans un craqueur propre à produire une coupe d'éthylène ou dans une unité autonome propre à produire une coupe d'éthylène.

12. Procédé selon l'une quelconque des revendications précédentes, dans lequel au moins une partie de la phase aqueuse (70) produite lors de l'étape de trempe/lavage est mise sous pression et est placée en contact avec le flux gazeux comprimé (76) lors de l'étape de lavage sous pression.

13. Procédé selon l'une quelconque des revendications précédentes, dans lequel au moins deux flux parmi le flux gazeux purifié (84), le solvant cryogénique hydrocarboné (85), le résidu gazeux léger (44), et/ou au moins un reflux ou un condensat de l'unité d'absorption cryogénique (42) sont placés en relation d'échange thermique dans une boite froide, en particulier formée d'un unique équipement cryogénique à plaques, notamment en aluminium brasé.

14. Installation (10 ; 110) de traitement d'au moins un flux gazeux (12) issu d'une pyrolyse de plastique et/ou de biomasse, **caractérisée par**:
- une unité (30) de trempe/lavage, propre à tremper et laver à l'eau le flux gazeux issu de la pyrolyse (12) de plastique et/ou de biomasse et à séparer une phase aqueuse (70) et un flux gazeux lavé (72);
- une unité (32) de compression/refroidissement du flux gazeux lavé (72) pour former un flux gazeux comprimé (76);
- une unité (34) de lavage sous pression du flux gazeux comprimé (76) pour former un flux gazeux lavé (78);
- au moins une unité (36, 38) d'élimination des acides présents dans le flux gazeux lavé (78) pour former un flux gazeux appauvri en acide (80);
- une unité (39) de séchage du flux gazeux appauvri en acide (80) pour former un flux gazeux sec (82);
- au moins une unité d'élimination d'impuretés (40) dans le flux gazeux sec (82) pour former un flux gazeux purifié (84) ;
- une unité d'absorption cryogénique (42) propre à être alimentée par le flux gazeux purifié (84) et par un solvant cryogénique hydrocarboné (85) pour obtenir, à une première sortie de l'unité d'absorption cryogénique (42), un résidu gazeux léger (44), et à une deuxième sortie de l'unité d'absorption cryogénique (42), une coupe (46) d'hydrocarbures en C₂⁺.

15. Installation (110) selon la revendication 14, dans laquelle une sortie de l'unité de trempe/lavage (30) récupérant la phase aqueuse (70) est raccordée à une entrée de l'unité de lavage sous pression (34), un organe (92) de compression de la phase aqueuse (70) étant interposé entre la sortie de l'unité de trempe/lavage (30) et l'entrée de l'unité de lavage haute pression (34).

16. Installation (10 ; 110) selon la revendication 14 ou 15, dans laquelle l'unité d'absorption cryogénique (42) comporte une boite froide, en particulier formée d'un unique équipement cryogénique à plaques, notamment en aluminium brasé, propre à placer en relation d'échange thermique au moins deux flux parmi le flux gazeux purifié (84), le solvant cryogénique hydrocarboné (85), le résidu gazeux léger (44), et/ou au moins un reflux ou un condensat de l'unité d'absorption cryogénique (42).

## Patentansprüche

1. Verfahren zum Behandeln mindestens eines Gasstroms (12), der aus einer Pyrolyse von Kunststoff und/oder Biomasse stammt,
**gekennzeichnet durch** die folgenden Schritte:
- Abschrecken und Waschen des Gasstroms, der aus der Pyrolyse (12) von Kunststoff und/oder Biomasse stammt, mit Wasser in einer Abschreck-/Wascheinheit (30) und Trennen einer wässrigen Phase (70) und eines gewaschenen Gasstroms (72);
- Komprimieren, dann Kühlen des gewaschenen Gasstroms (72), um einen komprimierten Gasstrom (76) auszubilden;
- Waschen des komprimierten Gasstroms (76) unter Druck, um einen gewaschenen Gasstrom (78) auszubilden;
- Leiten des gewaschenen Gasstroms (78) in mindestens eine Einheit (36, 38) zum Entfernen von Säuren, um einen an Säure abgereicherten Gasstrom (80) auszubilden;
- Trocknen des an Säure abgereicherten Gasstroms (80), um einen trockenen Gasstrom (82) auszubilden;
- Leiten des trockenen Gasstroms (82) in mindestens eine Einheit (40) zum Entfernen von Verunreinigungen, um einen gereinigten Gasstrom (84) auszubilden;
- Einleiten des gereinigten Gasstroms (84) in eine Einheit (42) für kryogene Absorption und Versorgen der Einheit (42) für kryogene Absorption mit kryogenem Kohlenwasserstofflösungsmittel (85), um an einem ersten Auslass der Einheit (42) für kryogene Absorption ein leichtes Restgas (44) und an einem zweiten Auslass der Einheit (42) für kryogene Absorption eine Fraktion (46) von C₂⁺-Kohlenwasserstoffen zu erhalten.

2. Verfahren nach Anspruch 1, wobei der Gasstrom, der aus der Pyrolyse (12) stammt, C₂⁺-Kohlenwasserstoffe und leichte Verbindungen wie Wasserstoff und Methan enthält.

3. Verfahren nach einem der Ansprüche 1 oder 2, wobei der Gasstrom, der aus der Pyrolyse (12) stammt, Kohlenmonoxid, Kohlendioxid, Stickoxide und/oder Sauerstoff und Sauerstoffderivate, insbesondere Alkohole, Aldehyde oder/und Ketone, Stickstoffverbindungen wie Stickstoff und Ammoniak und/oder saure Verbindungen wie Salzsäure und Blausäure, Chlorderivate und/oder Schwefelverbindungen, insbesondere Mercaptane, Kohlenstoffoxysulfide und Schwefelwasserstoff, enthält.

4. Verfahren nach einem der vorstehenden Ansprüche, wobei das kryogene Kohlenwasserstofflösungsmittel (85) C₃- und/oder C₄⁺-Kohlenwasserstoffe, insbesondere C₃-Kohlenwasserstoffe, umfasst.

5. Verfahren nach einem der vorstehenden Ansprüche, umfassend einen Schritt des Trennens der Fraktion (46) von C₂⁺-Kohlenwasserstoffen in eine Fraktion (14) von C₂-Kohlenwasserstoffen und in mindestens eine Fraktion (16, 18) von C₃⁺-Kohlenwasserstoffen, wobei mindestens ein Teil der Fraktion (16, 18) von C₃⁺-Kohlenwasserstoffen in die Einheit (42) für kryogene Absorption zurückgeführt wird.

6. Verfahren nach Anspruch 5, wobei der Trennschritt ein Erzeugen einer Fraktion (16) von C₃-Kohlenwasserstoffen und einer Fraktion (18) von C₄⁺-Kohlenwasserstoffen umfasst, wobei mindestens ein Teil der Fraktion (16) von C₃-Kohlenwasserstoffen in die Einheit (42) für kryogene Absorption zurückgeführt wird.

7. Verfahren nach einem der vorstehenden Ansprüche, wobei das leichte Restgas (44) Brenngas, insbesondere Methan, Wasserstoff sowie leichte Verbindungen des gereinigten Gasstroms (84), wie Kohlenmonoxid, Stickstoff, Stickstoffmonoxid und/oder Sauerstoff, enthält.

8. Verfahren nach einem der vorstehenden Ansprüche, wobei das leichte Restgas (44) dafür bestimmt ist, in eine Crackeinheit, insbesondere in das Brenngasnetz einer Dampfcrackeinheit, befördert zu werden und/oder dafür bestimmt ist, ein Kraftstoffgas zu bilden.

9. Verfahren nach einem der vorstehenden Ansprüche, wobei mindestens eine Einheit zum Entfernen von Säuren eine Amineinheit (36) oder/und eine kaustische Wascheinheit (38) umfasst, wobei der komprimierte Gasstrom (76) in die Amineinheit (36) oder/und in die kaustische Wascheinheit (38) geleitet wird.

10. Verfahren nach einem der vorstehenden Ansprüche, wobei die Einheit (40) zum Entfernen von Verunreinigungen mindestens ein Bett aus aktiviertem Metall, insbesondere ein Bett aus aktiviertem Aluminiumoxid oder einem gemischten Adsorptionsmittel, umfasst; wobei Schwefelverbindungen, insbesondere Sulfoxid von Wasserstoff und Mercaptanen, und/oder Ammoniak, Stickstoffverbindungen, Chlorverbindungen, sauerstoffhaltige Verbindungen, insbesondere Alkohole, und/oder Silikone, die in dem trockenen Gasstrom (82) enthalten sind, auf dem mindestens einen Bett aus aktiviertem Metall gehalten werden.

11. Verfahren nach einem der vorstehenden Ansprüche, wobei mindestens ein Teil der Fraktion (46) von C₂⁺-Kohlenwasserstoffen in einen Cracker, der geeignet ist, eine Ethylenfraktion zu erzeugen, oder in eine eigenständige Einheit, die geeignet ist, eine Ethylenfraktion zu erzeugen, eingeführt wird.

12. Verfahren nach einem der vorstehenden Ansprüche, wobei mindestens ein Teil der wässrigen Phase (70), die bei dem Abschreck-/Waschschritt erzeugt wird, unter Druck gesetzt wird und bei dem Waschschritt unter Druck mit dem komprimierten Gasstrom (76) in Kontakt gebracht wird.

13. Verfahren nach einem der vorstehenden Ansprüche, wobei mindestens zwei Ströme aus dem gereinigten Gasstrom (84), dem kryogenen Kohlenwasserstofflösungsmittel (85), dem leichten Restgas (44) und/oder mindestens ein Rückfluss oder ein Kondensat der Einheit (42) für kryogene Absorption in eine Wärmeaustauschbeziehung in einer Cold Box gebracht werden, die insbesondere aus einer einzigen kryogenen Plattenausrüstung, insbesondere aus gelötetem Aluminium, ausgebildet ist.

14. Anlage (10; 110) zum Behandeln mindestens eines Gasstroms (12), der aus einer Pyrolyse von Kunststoff und/oder Biomasse stammt, **gekennzeichnet durch:**
- eine Abschreck-/Wascheinheit (30), die dafür geeignet ist, den Gasstrom, der aus der Pyrolyse (12) von Kunststoff und/oder Biomasse stammt, mit Wasser abzuschrecken und zu waschen und eine wässrige Phase (70) und einen gewaschenen Gasstrom (72) zu trennen;
- eine Einheit (32) zum Komprimieren/Kühlen des gewaschenen Gasstroms (72), um einen komprimierten Gasstrom (76) auszubilden;
- eine Einheit (34) zum Waschen des komprimierten Gasstroms (76) unter Druck, um einen gewaschenen Gasstrom (78) auszubilden;
- mindestens eine Einheit (36, 38) zum Entfernen der Säuren, die in dem gewaschenen Gasstrom (78) vorhanden sind, um einen an Säure abgereicherten Gasstrom (80) auszubilden;
- eine Einheit (39) zum Trocknen des an Säure abgereicherten Gasstroms (80), um einen trockenen Gasstrom (82) auszubilden;
- mindestens eine Einheit (40) zum Entfernen von Verunreinigungen in dem trockenen Gasstrom (82), um einen gereinigten Gasstrom (84) auszubilden;
- eine Einheit (42) für kryogene Absorption, die geeignet ist, durch den gereinigten Gasstrom (84) und durch ein kryogenes Kohlenwasserstofflösungsmittel (85) versorgt zu werden, um an einem ersten Auslass der Einheit (42) für kryogene Absorption ein leichtes Restgas (44) und an einem zweiten Auslass der Einheit (42) für kryogene Absorption eine Fraktion (46) von C₂⁺-Kohlenwasserstoffen zu erhalten.

15. Anlage (110) nach Anspruch 14, wobei ein Auslass der Abschreck-/Wascheinheit (30), der die wässrige Phase (70) sammelt, mit einem Einlass der Wascheinheit (34) unter Druck verbunden ist, wobei ein Organ (92) zum Komprimieren der wässrigen Phase (70) zwischen dem Auslass der Abschreck-/Wascheinheit (30) und dem Einlass der Hochdruckwascheinheit (34) eingesetzt ist.

16. Anlage (10; 110) nach Anspruch 14 oder 15, wobei die Einheit (42) für kryogene Absorption eine Cold Box umfasst, die insbesondere aus einer einzigen kryogenen Plattenausrüstung, insbesondere aus gelötetem Aluminium, ausgebildet ist, die dafür geeignet ist, mindestens zwei Ströme aus dem gereinigten Gasstrom (84), das kryogene Kohlenwasserstofflösungsmittel (85), das leichte Restgas (44) und/oder mindestens einen Rückfluss oder ein Kondensat der Einheit (42) für kryogene Absorption in eine Wärmeaustauschbeziehung zu bringen.

## Claims

1. A method for treating at least one gas flow (12) resulting from a pyrolysis of plastic and/or of biomass, **characterized by** the following steps:
- tempering and washing the gas flow resulting from the pyrolysis (12) of plastic and/or of biomass in a tempering/washing unit (30) and separating an aqueous phase (70) and a washed gas flow (72);
- compressing, then cooling the washed gas flow (72) to form a compressed gas flow (76);
- pressurized washing of the compressed gas flow (76) to form a washed gas flow (78);
- passing the washed gas flow (78) into at least one acid removal unit (36, 38) to form an acid-depleted gas flow (80);
- drying the acid-depleted gas flow (80) to form a dry gas flow (82);
- passing the dry gas flow (82) into at least one impurity removal unit (40) to form a purified gas flow (84);
- introducing the purified gas flow (84) into a cryogenic absorption unit (42) and supplying the cryogenic absorption unit (42) with a cryogenic hydrocarbon solvent (85) to obtain, at a first outlet of the cryogenic absorption unit (42), a light gas residue (44), and at a second outlet of the cryogenic absorption unit (42), a cut (46) of C₂⁺ hydrocarbons.

2. The method according to claim 1, wherein the gas flow from the pyrolysis (12) contains C₂⁺ hydrocarbons, and light compounds such as hydrogen, and methane.

3. The method according to any one of claims 1 or 2, wherein the gas flow from the pyrolysis (12) contains carbon monoxide, carbon dioxide, nitrogen oxides, and/or oxygen and oxygen derivatives, in particular alcohols, aldehydes and/or ketones, nitrogen compounds such as nitrogen and ammonia and/or acid compounds, such as hydrochloric acid, and hydrocyanic acid, chlorine derivatives, and/or sulfur compounds, in particular mercaptans, carbon oxysulfides, and hydrogen sulfide.

4. The method according to any one of the preceding claims, wherein the cryogenic hydrocarbon solvent (85) comprises C₃ and/or C₄₊ hydrocarbons, in particular C₃ hydrocarbons.

5. The method according to any of the preceding claims, comprising a step of separating the cut (46) of C₂⁺ hydrocarbons into a cut (14) of C₂ hydrocarbons, and in at least one section (16, 18) of C₃⁺ hydrocarbons, at least part of said cut (16, 18) of C₃⁺ hydrocarbons being recycled into the cryogenic absorption unit (42).

6. The method according to claim 5, wherein the separation step comprises producing a cut (16) of C₃ hydrocarbons and a cut (18) of C₄⁺ hydrocarbons, at least part of the cut (16) of C₃ hydrocarbons being recycled into the cryogenic absorption unit (42).

7. The method according to any one of the preceding claims, wherein the light gas residue (44) contains combustible gas, in particular methane, hydrogen, as well as light compounds of the purified gas flow (84), such as carbon monoxide, nitrogen, nitrogen monoxide, and/or oxygen.

8. The method according to any one of the preceding claims, wherein the light gas residue (44) is intended to be conveyed in a cracking unit, in particular in the fuel gas network of a steam cracker and/or is intended to constitute a fuel gas.

9. The method according to any of the preceding claims, wherein at least one acid removal unit comprises an amine unit (36) or/and a caustic wash unit (38), the compressed gas flow (76) passing into the amine unit (36) or/and into the caustic wash unit (38).

10. The method according to any one of the preceding claims, wherein the impurity removal unit (40) comprises at least one activated metal bed, in particular an activated alumina bed or a mixed adsorbent; the sulfur compounds, in particular hydrogen oxysulfide and mercaptans, and/or ammonia, nitrogen compounds, chlorinated compounds, oxygenated compounds, in particular alcohols, and/or silicones contained in the dry gas flow (82) being retained on the at least one activated metal bed.

11. The method according to any of the preceding claims, wherein at least part of the cut (46) of C₂⁺ hydrocarbons is introduced into a cracker capable of producing an ethylene cut or in an autonomous unit capable of producing an ethylene cut.

12. The method according to any one of the preceding claims, wherein at least part of the aqueous phase (70) produced during the tempering/washing step is pressurized and is placed in contact with the compressed gas flow (76) during the pressure washing step.

13. The method according to any one of the preceding claims, wherein at least two of the purified gas flow (84), the cryogenic hydrocarbon solvent (85), the light gas residue (44), and/or at least one reflux or a condensate of the cryogenic absorption unit (42) are placed in a heat exchange relationship in a cold box, in particular one formed of a single cryogenic plate device, in particular made of brazed aluminum.

14. A facility (10; 110) for treating at least one gas flow (12) resulting from a pyrolysis of plastic and/or of biomass, **characterized by:**
- a tempering/washing unit (30), capable of tempering and washing the gas flow resulting from the pyrolysis (12) of plastic and/or of biomass and of separating an aqueous phase (70) and a washed gas flow (72);
- a unit (32) for compressing/cooling the washed gas flow (72) to form a compressed gas flow (76);
- a unit (34) for pressure-washing the compressed gas flow (76) to form a washed gas flow (78);
- at least one unit (36, 38) for removing acids present in the washed gas flow (78) to form an acid-depleted gas flow (80);
- a unit (39) for drying the acid-depleted gas flow (80) to form a dry gas flow (82);
- at least one unit (40) for removing impurities in the dry gas flow (82) to form a purified gas flow (84);
- a cryogenic absorption unit (42) able to be supplied by the purified gas flow (84) and by a cryogenic hydrocarbon solvent (85) to obtain, at a first outlet of the cryogenic absorption unit (42), a light gas residue (44), and at a second outlet of the cryogenic absorption unit (42), a cut (46) of C₂⁺ hydrocarbons.

15. The facility (110) according to claim 14, wherein an outlet of the tempering/washing unit (30) recovering the aqueous phase (70) is connected to an inlet of the pressurized washing unit (34), a member (92) for compressing the aqueous phase (70) being interposed between the outlet of the tempering/washing unit (30) and the inlet of the high-pressure washing unit (34).

16. The facility (10; 110) according to claim 14 or 15, wherein the cryogenic absorption unit (42) comprises a cold box, in particular one formed of a single cryogenic plate device, in particular made of brazed aluminum, able to place in a heat exchange relationship at least two flows from the purified gas flow (84), the cryogenic hydrocarbon solvent (85), the light gas residue (44), and/or at least one reflux or a condensate of the cryogenic absorption unit (42).
